# EUROPEAN PATENT SPECIFICATION

(11) **EP 3 310 349 B1**
(45) Date of publication and mention of the grant of the patent: **13.01.2021**
(21) Application number: 16734038.9
(22) Date of filing: 21.06.2016
(51) Int. Cl.: A61K 31/01, A61K 31/19, A61P 25/00

(54) **COMPOSITIONS AND METHODS FOR ENHANCING NEUROGENESIS IN ANIMALS**
ZUSAMMENSETZUNGEN UND VERFAHREN ZUR VERSTÄRKUNG DER NEUROGENESE IN TIEREN
COMPOSITIONS ET MÉTHODES POUR AUGMENTER LA NEUROGÉNÈSE CHEZ L'ANIMAL

(30) Priority: 22.06.2015 US 201562182922 P; 17.08.2015 US 201562205937 P
(43) Date of publication of application: 25.04.2018
(73) Proprietor: Société des Produits Nestlé S.A., 1800 Vevey (CH)
(72) Inventor: PAN, Yuanlong, Chesterfield, Missouri 63017 (US)
(74) Representative: Rupp, Christian
(86) International application number: PCT/IB2016/053689
(87) International publication number: WO 2016/207794

(56) References cited:
- WO-A1-2009/045481

## Description

### BACKGROUND OF THE INVENTION

### Field of the Invention

This invention relates generally to the neurological health of animals. In particular, the present invention relates to compositions and method for increasing neurogenesis in an animal.

### Description of Related Art

Neurogenesis at two locations in the brain of adult mammals, the subventricular zone (SVZ) of the lateral ventricles and subgranular zone (SGZ) of the dentate gyrus in the hippocampus, occurs throughout life and decline with aging (Cell 132: 645-660, 2008). Neural stem cells (NSCs) can differentiate into neurons, astrocytes and oligodendrocytes (Cell 132: 645-660, 2008). In the adult mammals, it is known that NSCs at SGZ of the dentate gyrus give rise to immature neurons which migrate into the inner granule cell layer differentiate into dentate granule cells in the hippocampus (Neuron 70: 687-702, 2011). A number of patent applications have been filed to promote neurogenesis at SVZ and SGZ. US Patent 8,383,626 teaches the art of using nitric oxide donors for inducing neurogenesis at SVZ and SGZ. EP 2314289 A1 provides composition and methods for treating diseases and conditions of central and peripheral nervous system by stimulating or increasing neurogenesis via a GABA agent. EP1940389 A2 describes composition and methods for treating diseases and conditions of central and peripheral nervous system by stimulating or increasing neurogenesis via inhibiting cyclic nucleotide phosphodiesterase. EP 2258358 A2 provides composition and methods for treating diseases and conditions of central and peripheral nervous system by stimulating or increasing neurogenesis via acetylcholinesterase inhibitors. WO 2010111136 A2 provides composition and methods for treating diseases and conditions of central and peripheral nervous system by stimulating or increasing neurogenesis via a renin inhibitor. A composition comprising one or more long chain fatty acids, nitric oxide releasing compounds, and medium chain triglycerides and methods for using such compositions for enhancing cognitive function, reducing or preventing a decline of social interaction, and reducing or preventing age-related behavioral changes is disclosed in WO 2009/045481 A1.

As such, it has been well established that new neurons are generated in the adult hippocampus throughout life by neural stem/progenitor cells (NSCs). Neurogenesis is a regenerative process responsive to external stimuli, involved in learning, memory and mood regulation. Neurogenesis is therefore believed to be an important form of neural regeneration, enabling organisms to adapt to environmental changes throughout life.

The rate of hippocampal neurogenesis significantly changes on physiological or pathological influences, such as physical activity, environmental enrichment, stress, mood disorders, ageing and neurodegenerative disorders. Indeed, during ageing, there is an age-related decline in adult neurogenesis. This decline is mostly related to decreased proliferation, associated to decreased stimulation to proliferate in an ageing brain. Natural or stimulated hippocampal neurogenesis play an important role in the clinical effects of antidepressants and possibly reduced neurogenesis may be involved in brain areas (including hippocampus) atrophy associated with depression. In Alzheimer's Disease (AD), there is also evidence for decreased neurogenesis, that accompanies the neuronal loss characteristic of the disease.

As such, there remains a need for compositions and methods to enhance neurogenesis in an animal.

### SUMMARY OF THE INVENTION

It is, therefore, an object of the present invention to provide methods useful for enhancing neurogenesis in a growing animal.

In one embodiment, a method for enhancing neurogenesis in a growing animal can comprise identifying the growing animal, and administering a composition comprising an unsaturated fatty acid (UFA), a nitric oxide releasing compound (NORC), a B vitamin, and an antioxidant, to the growing animal, wherein the composition is administered in an amount effective for enhancing neurogenesis in the growing animal. In another aspect, the instant invention provides a composition for use in enhancing neurogenesis in an animal that is suffering from cognitive decline, that is at risk of becoming cognitively impaired, that is diagnosed with a disease, disorder, or other condition that affects its cognitive abilities, or that is at risk of contracting a disease, disorder, or other condition that affects its cognitive abilities. Moreover, the composition of the present invention comprises an unsaturated fatty acid (UFA), a nitric oxide releasing compound (NORC), a B vitamin, and an antioxidant, in an amount effective for enhancing neurogenesis in the animal.

### DETAILED DESCRIPTION OF THE INVENTION

### Definitions

The following abbreviations may be used herein: AA, arachidonic acid; ALA, alpha-linolenic acid; DHA, docosahexaenoic acid; DPA, docosapentaenoic acid; EPA, eicosapentaenoic acid; LA, linoleic acid; UFA, unsaturated fatty acid; LCPUFA, long chain polyunsaturated fatty acid; NO, nitric oxide; NORC, nitric oxide releasing compound; and L-Arg, L-arginine.

The term "animal" refers to any animal that could benefit from one or more of the compositions for use according to the present invention or methods of the present invention including human, avian, bovine, canine, equine, feline, hircine, lupine, murine, ovine, and porcine animal. In one embodiment, the term "animal" means an animal for which an enhancement of neurogenesis is desired.

The term "companion animal" refers to any domesticated animal, and includes, without limitation, cats, dogs, rabbits, guinea pigs, ferrets, hamsters, mice, gerbils, horses, cows, goats, sheep, donkeys, pigs, and the like. In one example, the companion animal can be a dog or cat.

The term "B vitamin" refers to any B vitamin including derivatives, acidic forms, and salts thereof. Such B vitamins can include without limitation, vitamin B1 (thiamine), vitamin B2 (riboflavin), vitamin B3 (niacin, nicotinic acid, nicotinamide), vitamin B5 (pantothenic acid), vitamin B6 (pyridoxine, pyridoxal, pyridoxamine), vitamin B7 (biotin), vitamin B8 (myo-inositol), vitamin B9 (folic acid) vitamin B12 (cobalamin compounds including methylcobalamin, hydroxocobalamin, and cyanocobalamin).

The term "unsaturated fatty acid" or "UFA" refers to polyunsaturated fatty acids or monounsaturated fatty acids, including monocarboxylic acids having at least one double bond. UFAs include (n-6) fatty acids such as linoleic acid (LA) and arachidonic acid (AA) and (n-3) fatty acids such as eicosapentaenoic acid (EPA), alpha-linolenic acid (ALA), docosapentaenoic acid (DPA) and docosahexaenoic acid (DHA). UFAs also include myristoleic acid, palmitoleic acid, oleic acid, linoleic acid, cis-vaccenic acid, and erucic acid.

The term "fish oil" refers to a fatty or oily extract, relatively rich in UFA, whether crude or purified, obtained from a sea animal, such as a cold-water fish including, but not limited to, salmon, tuna, mackerel, herring, sea bass, striped bass, halibut, catfish, and sardines, as well as shark, shrimp, and clams, or any combination thereof. Fish oil is generally a term of art used by ingredient suppliers and encompasses a range of products of varying UFA content and purity.

The term "nitric oxide releasing compound" or "NORC" refers to any compound or compounds that cause or can result in the release of nitric oxide in an animal. Examples of such compounds include L-arginine, L-arginine-containing peptides and proteins, and analogs or derivatives thereof that are known or determined to release nitric oxide, such as arginine alpha-ketoglutarate, GEA 3175, sodium nitroprusside, glyceryl trinitrate, S-nitroso-N-acetyl-penicillamine, nitroglycerin, S-NO-glutathione, NO-conjugated non-steroidal anti-inflammatory drugs (e.g., NO-naproxen, NO-aspirin, NO-ibuprofen, NO-Diclofenac, NO-Flurbiprofen, and NO-Ketoprofen), NO-releasing compound-7, NO-releasing compound-5, NO-releasing compound-12, NO-releasing compound-18, diazenium diolates and derivatives thereof, diethylamine NONOate, and any organic or inorganic compound, biomolecule, or analog, homolog, conjugate, or derivative thereof that causes the release of nitric oxide, particularly "free" NO, in an animal. NORC is also defined to include supplements that can be converted to nitric oxide releasing compounds when metabolized in the body, e.g., citrulline and ornithine.

The term "recommended daily requirement" or "recommended daily allowance" or "RDA" refers to the daily amount of a vitamin or other nutrient recommended by a recognized agency or standard within the art including without limitation, National Institutes of Health (NIH), Institute of Medicine of the National Academies (IOM), Dietary Reference Intake (DRI) Nutrient Report, The European Pet Food Industry Federation (FEDIAF), and The Association of American Feed control Officials (AAFCO) as of January 1, 2015.

The term "aging" means being of advanced age such that the animal has exceeded 50% of the average lifespan for its particular species and/or breed within a species. For example, if the average lifespan for a given breed of dog is 10 years, then a dog within that breed greater than 5 years old would be considered "aging" for purposes herein. "Healthy aging animals" are those with no known diseases, particularly diseases relating to loss of cognitive impairment such as might confound the results. In studies using healthy aging animals, cohort animals are also healthy aging animals, although other healthy animals with suitable cognitive, motor, or behavioral functioning may be suitable for use as comparative specimens. If animals with specific disease diagnoses, or cognitive, motor, or behavioral limitations are used, then the cohort animals should include animals that are similarly diagnosed, or which present with similar indicia of the disease or cognitive, motor, or behavioral limitation.

The term "long-term administration" means periods of repeated administration or consumption in excess of one month. Periods of longer than two, three, or four can be used for certain embodiments. Also, more extended periods that include longer than 5, 6, 7, 8, 9, or 10 months can be used. Periods in excess of 11 months or 1 year can also be used. Longer term use extending over 1, 2, 3, or more years can also be included in the invention. For certain aging animals, the animal will continue consuming on a regular basis for the remainder of its life. Sometimes this is referred to as consumption for "extended" periods.

The term "regular basis" means at least monthly dosing with the compositions or consumption of the compositions, and in some aspects, weekly dosing. More frequent dosing or consumption, such as twice, three, or seven times weekly, can be used in certain embodiments. Still other embodiments include regimens that comprise at least once daily consumption. The skilled artisan will appreciate that the blood level of a compound or certain metabolites of that compound or which result after the consumption of that compound, may be a useful tool for assessing or determining dosing frequency. A frequency, regardless of whether expressly exemplified herein, that allows maintenance of a desired blood level of the measured compound within acceptable ranges is useful herein. The skilled artisan will appreciate that dosing frequency will be a function of the composition that is being consumed or administered, and some compositions may require more or less frequent administration to maintain a desired blood level of the measured compound.

The term "about" includes all values within a range of 5% of the stated number. In one embodiment, "about" includes all values within a range of 2%, and in one aspect, within 1 %.

The term "individual" when referring to an animal means an individual animal of any species or kind.

As used throughout, ranges are used herein in shorthand, so as to avoid having to set out at length and describe each and every value within the range. Any appropriate value within the range can be selected, where appropriate, as the upper value, lower value, or the terminus of the range.

As used herein, embodiments, aspects, and examples using "comprising" language or other open-ended language can be substituted with "consisting essentially of' and "consisting of' embodiments.

As used herein and in the appended claims, the singular form of a word includes the plural, and vice versa, unless the context clearly dictates otherwise. Thus, the references "a", "an", and "the" are generally inclusive of the plurals of the respective terms. For example, reference to "a dog" or "a method" includes a plurality of such "dogs" or "methods". Reference herein, for example to "an unsaturated fatty acid" includes a plurality of such unsaturated fatty acids, whereas reference to "B vitamins" includes a single B vitamin. Similarly, the words "comprise", "comprises", and "comprising" are to be interpreted inclusively rather than exclusively. Likewise the terms "include", "including" and "or" should all be construed to be inclusive, unless such a construction is clearly prohibited from the context. Where used herein the term "examples," particularly when followed by a listing of terms is merely exemplary and illustrative, and should not be deemed to be exclusive or comprehensive.

The methods and compositions and other advances disclosed here are not limited to particular methodology, protocols, and reagents described herein because, as the skilled artisan will appreciate, they may vary. Further, the terminology used herein is for the purpose of describing particular embodiments only, and is not intended to, and does not, limit the scope of that which is disclosed or claimed.

Unless defined otherwise, all technical and scientific terms, terms of art, and acronyms used herein have the meanings commonly understood by one of ordinary skill in the art in the field(s) of the invention, or in the field(s) where the term is used. Although any compositions, methods, articles of manufacture, or other means or materials similar or equivalent to those described herein can be used in the practice of the present invention, certain compositions, methods, articles of manufacture, or other means or materials are described herein.

.

### The Invention

The present inventors have discovered that certain compositions can enhance neurogenesis in an animal. In one embodiment, a method for enhancing neurogenesis in an growing animal or a composition for use in enhancing neurogenesis in an animal can comprise administering a composition comprising an unsaturated fatty acid (UFA), a nitric oxide releasing compound (NORC), a B vitamin, and an antioxidant, to the animal, wherein the composition is administered in an amount effective for enhancing neurogenesis in the animal. In one embodiment, the antioxidant and B vitamin are administered above RDA values. In one aspect, the antioxidant and the B vitamin can be administered at least 1.2 times the RDA value.

In one aspect, the neurogenesis can occur at, but not limited to, hippocampus in an animal. In one aspect, the neurogenesis can occur at the CA1 region of the hippocampus. Such neurogenesis can be measured as an increase of neurons as compared to a comparable animal at the same stage of life. In one aspect, the present methods or compositions for use according to the present invention can provide at least a 10% increase in neurons. In another aspect, the present methods or compositions for use according to the present invention can provide at least 20%, 30%, or even 40% increase in neurons.

In one embodiment, the present methods can include identifying a growing animal. In one aspect, the growing animal can be an infant, a kitten, or a puppy or any young animal that has not reached its full size (height) and adult weight. In another aspect, a growing animal can be an animal that has reached between 0 and 30% of its life expectancy.

, Methods can include identifying an animal that is suffering from cognitive decline or that is at risk of becoming cognitively impaired. The methods can include an animal that is diagnosed with a disease, disorder, or other condition that affects its cognitive abilities or that is at risk of contracting a disease, disorder, or other condition that affects its cognitive abilities. Determining an animal that is at risk for cognitive decline or at risk for contracting a disease, disorder, or other condition that affects cognitive abilities can include, without limitation, animals with a family history of cognitive decline or disease, disorder, or other condition that affects cognitive abilities and animals having a genetic predisposition of cognitive decline or disease, disorder, or other condition that affects cognitive abilities.

The UFA can be any UFA suitable for administration to an animal. UFAs can be obtained from any suitable source, synthetic or natural. Sources of UFA include natural sources of such fatty acids and include, without limitation, primrose; dark green vegetables such as spinach; algae and blue-green algae such as spirulina; plant seeds and oils from plants such as flax, canola, soybean, walnut, pumpkin, safflower, sesame, wheat germ, sunflower, corn, and hemp; and fish such as salmon, tuna, mackerel, herring, sea bass, striped bass, halibut, catfish, sardines, shark, shrimp, and clams; and the extracted oils of any one or more of the foregoing. The UFA may also be synthetic, and as such may be produced according to any means suitable in the art, from any suitable starting material. The UFA may comprise a blend of any one or more UFA from any one or more sources, such as those exemplified above, whether natural or synthetic. In some embodiments, the UFA can be long chain polyunsaturated fatty acids (LCPUFA) comprising one or more monocarboxylic acids having at least 20 carbon atoms and at least two double bonds. In one aspect, the UFAs can be (n-6) fatty acids or (n-3) fatty acids, and in specific aspects, the UFAs can be n-3 LCPUFAs.

The NORC can be any NORC suitable for administration to an animal. NORC can be obtained from any suitable source, synthetic or natural. In various embodiments, the NORC comprises arginine. Sources of arginine include, without limitation, animal and plant proteins. Examples of plants considered rich in arginine content and suitable for use herein include, but are not limited to, legumes such as soy, lupins, and carob; grains such as wheat and rice; and fruits such as grapes. Seeds and nuts of plants such as cacao and peanut are also considered rich in arginine content and are therefore useful herein. Some examples of suitable animal proteins considered rich in arginine content are poultry and fish products. The NORC can also be synthetically produced, according to any suitable means in the art. As with UFA, the NORC content of any composition disclosed herein can include a blend of any natural or synthetic NORC. Both UFA and NORC, whether natural or synthetic, can be obtained directly or provided by a commercial source.

The B vitamins can be any B vitamin suitable for administration to an animal. B vitamins include vitamin B1 (thiamine), vitamin B2 (riboflavin), vitamin B3 (niacin, nicotinic acid, nicotinamide), vitamin B5 (pantothenic acid), vitamin B6 (pyridoxine, pyridoxal, pyridoxamine), vitamin B7 (biotin), vitamin B8 (myo-inositol), vitamin B9 (folic acid) vitamin B12 (cobalamin compounds including methylcobalamin, hydroxocobalamin, and cyanocobalamin), or salts, conjugates, or derivatives thereof recognized of found to have B vitamin activity. Combinations of any of the foregoing are also useful herein and are sometimes referred to herein as "mixtures" of B vitamins. Since the vitamin requirements vary for different species, not all of the listed compounds are deemed vitamins for all species. For example, since it is known that myo-inositol can be synthesized by humans, it is no longer deemed a vitamin, as it is not required for adequate human nutrition.

The antioxidants can be any antioxidant suitable for administration to an animal. Antioxidants are well known in the art, particularly the art of food technology and food formulation. Natural antioxidant compounds include vitamins (such as A, C and E, and derivative, conjugates, or analogs thereof), as well as plant extracts, including extracts from fruit, vegetables, herbs, seeds, and other types and/or parts of plants. Compounds such as α-lipoic acid, chlorophyll and derivatives thereof, glutathione, ubiquinols (*e.g.,* coenzyme Q10), carotenoids (*e.g.*, beta-carotene, alpha-carotene, beta-cryptoxanthin, gamma-carotene, lutein, astaxanthin, and zeaxanthin etc.), flavonoids, phenolic acids and polyphenols, and pycnogenol are known to be excellent antioxidants. Some examples of plant sources of antioxidants include those from fruits such as berries (cherry, blackberry, strawberry, raspberry, crowberry, blueberry, bilberry/wild blueberry, black currant), pomegranate, grape, orange, plum, pineapple, kiwi fruit, and grapefruit; those from vegetables including kale, chili pepper, red cabbage, peppers, parsley, artichoke, Brussels sprouts, spinach, lemon, ginger, garlic, and red beets; those from dry fruits like apricots, prunes, and dates; from legumes including broad beans, pinto beans, and soybeans. Also nuts and seeds such as pecans, walnuts, hazelnuts, ground nut, and sunflower seeds; cereals such as barley, millet, oats, and corn. Many natural antioxidants are also available from a wide variety of spices including cloves, cinnamon, rosemary, and oregano. Less widely known sources of antioxidants include *Ginkgo biloba,* and tropical plants such as uyaku, and carica papaya. Antioxidant properties of various teas and green tea, as well as fermented products such as red wine, have become of great interest in recent years and such would be suitable for use herein. Selenium is an excellent oxygen scavenger and works well, especially with vitamin or related tocopherol compounds. Synthetic dietary antioxidants include butylated hydroxyanisole (BHA) and butylated hydroxytoluene (BHT) which are commonly used in food products. Any of the foregoing, alone or in combination, are suited for use herein, as are combinations of natural and synthetic antioxidants. In one embodiment, the antioxidants comprise astaxanthin alone or in combination with other antioxidants.

The compositions comprise UFA and NORC, B vitamins and antioxidants, in an amount effective for enhancing neurogenesis. Generally, the compositions comprise from about 0.1% to about 50% UFA, and from about 0.1% to about 20% NORC. Additionally, the composition comprises from about 1.2 to 40 times the recommended daily requirement (RDA) of B vitamins and from about 0.0001% to about 25% of antioxidants. In various embodiments, the compositions comprise from about 1 to about 30% UFA, and in one aspect, from about 1 to about 15% UFA; and from about 1 to about 15% NORC, and in one aspect, from about 1 to about 10% NORC. In various embodiments, the B vitamins comprise from about 1.2 to 20 times the RDA, and in one aspect, from about 1.2 to 10 times the RDA, and antioxidants comprise from about 0.0001% to about 15%, in one aspect, from about 0.001% to about 5%, and in one specific aspect, from about 0.001% to about 2%. In one embodiment, the composition comprises from about 0.5g to about 10g UFA, and from about 0.5g to about 10g NORC, with 1.5-4 times the RDA for B vitamins and antioxidants. In some aspects, the composition can include at least 4 times the RDA for B vitamins.

The compositions may further comprise substances such as minerals, other vitamins, salts, nutrients, functional additives including, for example, palatants, colorants, emulsifiers, antimicrobial or other preservatives. Minerals that may be useful in such compositions include, for example, calcium, phosphorous, potassium, sodium, iron, chloride, boron, copper, zinc, magnesium, manganese, iodine, selenium and the like. Examples of additional vitamins useful herein include such fat soluble vitamins as D and K. Inulin, amino acids, taurine, choline, enzymes, coenzymes, and the like may be useful to include in various embodiments.

In one embodiment, the compositions are food compositions, including human and pet food compositions. Such compositions include foods intended to supply the necessary dietary requirements for an animal, animal treats (*e.g.,* biscuits), or dietary supplements. The compositions may be a dry composition (*e.g.,* kibble), semi-moist composition, wet composition, or any mixture thereof. In another embodiment, the composition is a dietary supplement such as a gravy, drinking water, beverage, yogurt, powder, granule, paste, suspension, chew, morsel, treat, snack, pellet, pill, capsule, tablet, or any other suitable delivery form. The dietary supplement can comprise a high concentration of the UFA and NORC, and B vitamins and antioxidants. This permits the supplement to be administered to the animal in small amounts, or in the alternative, can be diluted before administration to an animal. The dietary supplement may require admixing, or can be admixed with water or other diluent prior to administration to the animal.

In one embodiment, the compositions can be refrigerated or frozen compositions. In another embodiment, the UFA and NORC can be pre-blended with the other components to provide the beneficial amounts needed. In yet other embodiments, the UFA and NORC can be used to coat a food, snack, pet food composition, or pet treat. In one embodiment, the UFA and NORC can be added to the composition just prior to offering it to the animal, *e.g.,* using a sprinkled powder or a mix. Such compositions further comprise B vitamins and/or antioxidants.

The compositions can optionally comprise one or more supplementary substances that promote or sustain general health. Preferred substances may be associated with improved mental health or enhanced cognitive function or may be substances that inhibit, delay, or decrease loss of cognitive function, *e.g.,* herbs or plants that enhance cognitive function.

In various embodiments, pet food or pet treat compositions comprise from about 15% to about 50% crude protein. The crude protein material may comprise vegetable proteins such as soybean meal, soy protein concentrate, corn gluten meal, wheat gluten, cottonseed, and peanut meal, or animal proteins such as casein, albumin, and meat protein. Examples of meat protein useful herein include pork, lamb, equine, poultry, fish, and mixtures thereof.

The compositions may further comprise from about 5% to about 40% fat. The compositions may further comprise a source of carbohydrate. The compositions may comprise from about 15% to about 60% carbohydrate. Examples of such carbohydrates include grains or cereals such as rice, corn, milo, sorghum, alfalfa, barley, soybeans, canola, oats, wheat, and mixtures thereof. The compositions may also optionally comprise other materials such as dried whey and other dairy by-products.

In some embodiments, the ash content of the composition ranges from less than 1% to about 15%, and in one aspect, from about 5% to about 10%.

The moisture content can vary depending on the nature of the composition. In a one embodiment, the composition can be a complete and nutritionally balanced pet food. In this embodiment, the pet food may be a "wet food", "dry food", or food of intermediate moisture content. "Wet food" describes pet food that is typically sold in cans or foil bags, and has a moisture content typically in the range of about 70% to about 90%. "Dry food" describes pet food which is of a similar composition to wet food, but contains a limited moisture content, typically in the range of about 5% to about 15% or 20%, and therefore is presented, for example, as small biscuit-like kibbles. In one embodiment, the compositions have moisture content from about 5% to about 20%. Dry food products include a variety of foods of various moisture contents, such that they are relatively shelf-stable and resistant to microbial or fungal deterioration or contamination. Also included are dry food compositions which are extruded food products, such as pet foods, or snack foods for either humans or companion animals.

The compositions may also comprise one or more fiber sources. The term "fiber" includes all sources of "bulk" in the food whether digestible or indigestible, soluble or insoluble, fermentable or nonfermentable. Fibers can be from plant sources such as marine plants but microbial sources of fiber may also be used. A variety of soluble or insoluble fibers may be utilized, as will be known to those of ordinary skill in the art. The fiber source can be beet pulp (from sugar beet), gum arabic, gum talha, psyllium, rice bran, carob bean gum, citrus pulp, fructooligosaccharide, pectin, short chain oligofructose, mannanoligofructose, soy fiber, arabinogalactan, galactooligosaccharide, arabinoxylan, or mixtures thereof.

Alternatively, the fiber source can be a fermentable fiber. Fermentable fiber has previously been described to provide a benefit to the immune system of a companion animal. Fermentable fiber or other compositions known to skilled artisans that provide a prebiotic to enhance the growth of probiotics within the intestine may also be incorporated into the composition to aid in the enhancement of the benefit provided by the present invention to the immune system of an animal.

In other embodiments, the compositions further comprise prebiotics, probiotics, or a combination thereof. Probiotics are live microorganisms that have a beneficial effect in the prevention and treatment of specific medical conditions when ingested. Probiotics are believed to exert biological effects through a phenomenon known as colonization resistance. The probiotics facilitate a process whereby the indigenous anaerobic flora limits the concentration of potentially harmful (mostly aerobic) bacteria in the digestive tract. Other modes of action, such as supplying enzymes or influencing enzyme activity in the gastrointestinal tract, may also account for some of the other functions that have been attributed to probiotics. Prebiotics are nondigestible food ingredients that beneficially affect host health by selectively stimulating the growth and/or activity of bacteria in the colon. Prebiotics include fructooligosaccharides (FOS), xylooligosaccharides (XOS), galactooligosaccharides (GOS), and mannooligosaccharides (typically for non-human foods such as petfoods). The prebiotic, fructooligosaccharide (FOS) can be found naturally in many foods such as wheat, onions, bananas, honey, garlic, and leeks. FOS can also be isolated from chicory root or synthesized enzymatically from sucrose. FOS fermentation in the colon results in a large number of physiologic effects including increasing the numbers of bifidobacteria in the colon, increasing calcium absorption, increasing fecal weight, shortening of gastrointestinal transit time, and possibly lowering blood lipid levels. The increase in bifidobacteria has been assumed to benefit human health by producing compounds to inhibit potential pathogens, by reducing blood ammonia levels, and by producing vitamins and digestive enzymes. Probiotic bacteria such as Lactobacilli or Bifidobacteria are believed to positively affect the immune response by improving the intestinal microbial balance leading to enhanced antibody production and phagocytic (devouring or killing) activity of white blood cells. Bifidobacterium lactis could be an effective probiotic dietary supplement for enhancing some aspects of cellular immunity in the elderly. Probiotics enhance systemic cellular immune responses and may be useful as a dietary supplement to boost natural immunity in otherwise healthy adults. Probiotics include many types of bacteria but generally are selected from four genera of bacteria: *Lactobacilllus acidophillus, Bifidobacteria, Lactococcus,* and *Pediococcus.* Beneficial species include *Enterococcus* and *Saccharomyces* species. The amount of probiotics and prebiotics to be administered to the animal is determined by the skilled artisan based upon the type and nature of the prebiotic and probiotic and the type and nature of the animal, e.g., the age, weight, general health, sex, extent of microbial depletion, presence of harmful bacteria, and diet of the animal. Generally, probiotics are administered to the animal in amounts of from about one to about twenty billion colony forming units (CFUs) per day for the healthy maintenance of intestinal microflora, preferably from about 5 billion to about 10 billion live bacteria per day. Generally, prebiotics are administered in amounts sufficient to positively stimulate the healthy microflora in the gut and cause these "good" bacteria to reproduce. Typical amounts are from about one to about 10 grams per serving or from about 5% to about 40% of the recommended daily dietary fiber for an animal. The probiotics and prebiotics can be made part of the composition by any suitable means. Generally, the agents are mixed with the composition or applied to the surface of the composition, *e.g.,* by sprinkling or spraying.

The compositions and dietary supplements may be specially formulated for the intended recipients or consumers, such as for adult animals or for older or young animals. For example, a composition adapted for puppies or kittens or adapted for active, pregnant, lactating, or aging animals can be prepared. In general, specialized compositions will comprise energy and nutritional requirements appropriate for animals at different stages of development or age.

Certain aspects of the invention can be used in combination with a complete and balanced food. According to certain embodiments provided herein, the compositions comprising the UFA, NORC, B vitamins, and antioxidants, can be used with a high-quality commercial food. As used herein, "high-quality commercial food" refers to a diet manufactured to produce the digestibility of the key nutrients of 80% or more, as set forth in, for example, the recommendations of the National Research Council above for dogs, or in the guidelines set forth by the Association of American Feed Control Officials. Similar high nutrient standards would be used for other animals.

In one embodiment, the food compositions comprise any of a variety of ingredients or combinations thereof selected for their contributions to the overall composition. Thus a skilled food technologist may choose from among natural (*e.g.,* plant or plant-derived, animal or animal-derived, and microbial or microbially-derived), and synthetic ingredients or components. In particular embodiments, the ingredients may include any of the cereal grains and/or fractions or components thereof, meat and meat by-products, fish, shellfish, or other seafood, other animal products or by-products, eggs from any source, vitamins, minerals, salts, sweeteners, fiber, flavoring or other palatants, coloring, and functional ingredients such as emulsifiers, stabilizers, softeners, functional coatings, and the like. Cereals useful in the invention include all plants recognized as "cereal" crops, whether currently used in commercial agriculture or merely known practically or botanically as being a "cereal". For example, "cereals" includes corn, wheat, rice, barley, sorghum, millet, oats, rye, triticale, buckwheat, fonio, and quinoa. The skilled artisan will appreciate that in a given food composition, it is not uncommon to use one or more such cereal products. Meats useful in the invention include products from any animal, including muscle tissue such as chicken or other poultry, lamb, sheep, veal, beef, or pork. Other animal products and by-products useful in the invention include dairy products or by-products derived from the milk of any species. Other important components or ingredients include fats and the skilled artisan will appreciate that many sources of vegetable, animal, or microbial fats are available for formulating food compositions. In one embodiment, the source of fat is a plant fat such as corn, soy, or canola oil, including those that are readily available. In another embodiment, an animal fat, such as tallow, is useful for providing calories from fat, as well as enticing flavor to meat-eating animals. Of course, combinations of any of the foregoing ingredients, such as fats, are known in the art and useful for optimizing the food compositions based on functional properties as well as price and availability.

The skilled artisan will also appreciate that in formulating the food compositions of the invention, the formulation may vary slightly, so as to allow consideration by the formulator of the price and/or availability of certain ingredients in the compositions, as well as the batch-to-batch variation in the analysis of certain ingredients. Thus a given food composition or formulation may vary slightly from batch to batch, plant to plant, or even season to season depending on such factors. Notwithstanding such variation in specific ingredients selected for manufacturing a particular batch of a food composition, the overall composition (for example, analysis of protein, carbohydrate, fat, fiber, or other component) may be held constant or at least substantially constant, for example, in accordance with a label claim, such as a claim or guarantee of a minimum or maximum percent of a particular component.

For pet foods and food products formulated for human consumption, the amount of UFA as a percentage of the composition can be in the range of about 0.1% to about 13% of the composition, although a greater percentage can be supplied. In various embodiments, the amount of UFA can be about 0.1%, 0.2%, 0.3%, 0.4%, 0.5%, 0.6%, 0.7%, 0.8%, 0.9%, 1.0%, 1.1%, 1.2%, 1.3%, 1.4%, 1.5%, 1.6%, 1.7%, 1.8%, 1.9%, 2.0%, 2.1%, 2.2%, 2.3%, 2.4%, 2.5%, 2.6%, 2.7%, 2.8%, 2.9%, 3.0%, 3.1%, 3.2%, 3.3%, 3.4%, 3.5%, 3.6%, 3.7%, 3.8%, 3.9%, 4.0%, 4.1%, 4.2%, 4.3%, 4.4%, 4.5%, 4.6%, 4.7%, 4.8%, 4.9%, 5.0%, or more, e.g., 6%, 7%, 8%, 9%, 10%, 11%, 12%, 13%, 14%, 15%, 16%, 17%, 18%, 19%, 20% or more, of the composition. Up to 30, 40, or 50 % UFA may be used in certain embodiments.

For pet foods and food products formulated for human consumption, the amount of NORC as a percentage of the composition can be in the range of about 0.1% to about 12% of the composition, although a greater percentage can be supplied. In various embodiments, the amount of NORC can be about 0.1%, 0.2%, 0.3%, 0.4%, 0.5%, 0.6%, 0.7%, 0.8%, 0.9%, 1.0%, 1.1%, 1.2%, 1.3%, 1.4%, 1.5%, 1.6%, 1.7%, 1.8%, 1.9%, 2.0%, 2.1%, 2.2%, 2.3%, 2.4%, 2.5%, 2.6%, 2.7%, 2.8%, 2.9%, 3.0%, 3.1%, 3.2%, 3.3%, 3.4%, 3.5%, 3.6%, 3.7%, 3.8%, 3.9%, 4.0%, 4.1%, 4.2%, 4.3%, 4.4%, 4.5%, 4.6%, 4.7%, 4.8%, 4.9%, 5.0%, or more, *e.g.,* 6%, 7%, 8%, 9%, 10%, 11%, 12% or more, up to about 15% or even 20% of the composition. In specific embodiments, 2-2.5% UFA and 2-2.5% NORC can be used. In such embodiments, pure L-arginine can be the NORC compound. Dietary supplements may be formulated to contain several-fold higher concentrations of UFA and NORC, to be amenable for administration to an animal in the form of a tablet, capsule, liquid concentrate, emulsion, suspension, gel, or other dosage form, or to be diluted before administration, such as by dilution in water, or adding to a pet food (for example, by spraying or sprinkling thereon), and other modes of administration suitable for administering such dietary supplements.

In an alternative embodiment, the amount of UFA and NORC in the composition can be a function of an amount required to establish specified concentrations, or a desired range of concentrations, of UFA and/or NORC, or a metabolite thereof, in the blood serum of the animal. The specified concentrations, or desired ranges of UFA and/or NORC in the blood serum may be calculated by determining the blood serum levels of animals fed the recommended amounts of UFA and NORC specified above, as would be appreciated by one of skill in the art.

In one embodiment, the food compositions comprise a macronutrient composition suitable for the type of food being designed. In one embodiment, the food composition can have about 20 to 32% protein, about 30 to 50% carbohydrate, about 5% to 20% fat, and about 15% to 25% moisture. In another embodiment, the food composition can be a pet food composition such as a premium or super-premium pet food composition. In one embodiment, the pet food can be formulated for canines and has a protein content of about 20-30%, in one aspect, about 24-28%, and in one specific aspect, about 25-27%. In one embodiment, the protein content of a dog food composition can be about 26% by weight. In another embodiment, the formulation can be for felines and can have a protein content of about 35-45%, in one aspect, about 37-42%, and in one specific aspect, about 39-41%. In one embodiment, the protein content of a cat food composition can be about 40%. In another embodiment, the composition can be a food product comprising UFA, NORC, B vitamins, and antioxidants, and further comprising about 15% to about 50% protein, about 5% to about 40% fat, about 5% to about 10% ash content, and having a moisture content of about 5% to about 20%.

In one embodiment, the food composition can be a wet food, such as a canned food, frozen food, or fresh food product. In one embodiment, the food composition can be shelf stable. In another, it can be refrigerated. In other embodiments, the food composition can be an intermediate moisture product or a dry food product as described above.

In one embodiment, the UFA can be a fish oil and the NORC can be arginine or a nitric oxide-releasing derivative thereof. In certain embodiments, the compositions can comprise from about 0.1% to about 50% fish oil and from about 0.1% to about 20% arginine. In some embodiments, both UFA and NORC can be within the ranges provided herein when the composition is a food product. Such ranges can offer improved palatability and functional properties that will enhance acceptance of the food product.

In one embodiment, the composition can be formulated for a companion animal, *e.g.,* a dog or cat. In other embodiments, the animal is a human, with or without an age-related cognitive decline.

In one embodiment, the composition can be formulated to provide about 0.5g to about 10g each of UFA and NORC per day in one or more portions of a recommended serving size. For example, if the composition is intended for twice-per-day consumption, a 10g dose can be provided by formulating the composition such that each recommended portion (*e.g.,* 2 spoonsful, or one 20g portion, or the like) to provide 5g each of UFA and NORC.

In one embodiment, the compositions can comprise from about 0.1% to about 50% fish oil, from about 0.1% to about 20% arginine, 4 to 40 times the recommended daily requirement of B vitamins, and from about 0.0001% to about 25% antioxidants. In one embodiment, the composition comprises from about 0.5g to about 10g UFA, and from about 0.5g to about 10g NORC, with B vitamins and antioxidants.

In another aspect, the compositions further comprise one or more cognitive drugs in an amount effective for enhancing cognitive function. The skilled artisan can determine the amount of cognitive drug to be added to the composition based upon the recommended dosage for the drug given by its manufacturer or upon the animal's weight, species, age, health status, and the like.

In another aspect, the invention provides pharmaceutical compositions comprising a composition of the present invention and one or more pharmaceutically-acceptable carriers, diluents, or excipients. Generally, pharmaceutical compositions are prepared by admixing a compound or composition with excipients, buffers, binders, plasticizers, colorants, diluents, compressing agents, lubricants, flavorants, moistening agents, and the like, including other ingredients known to skilled artisans to be useful for producing pharmaceuticals and formulating compositions that are suitable for administration to an animal as pharmaceuticals.

In some embodiments, the UFA, NORC, B vitamins, and antioxidants can be administered to the animal in amounts given herein when describing the compositions. In certain embodiments, the daily dose for the compositions can range from about 5 mg/day to about 5,000 mg/day, 10,000 mg/day, or 20,000, or more per animal. In one aspect, the daily dose ranges from about 30 mg/day to about 10,000 mg/day per animal, and in another aspect, from about 750 mg/day to about 7,500 mg/day per animal. The daily dose of UFA and NORC can be measured in terms of grams of UFA and NORC per kg of body weight (BW) of the animal. The daily dose of UFA and NORC, therefore can range from about 0.001 g/kg to about 50 g/kg BW of the animal, although greater or lesser doses can be provided. In one aspect, the daily dose of UFA and NORC can be from about 0.001g/kg to about 25 g/kg BW of the animal. In another aspect, the daily dose of UFA and NORC can be from about 0.001 g/kg to about 10 g/kg BW of the animal. In other aspects, the daily dose of UFA and NORC can be from about 0.001 g/kg to about 5 g/kg BW of the animal, from about 0.001 g/kg to about 1 g/kg BW of the animal, or even from about 0.001 g/kg to about 0.5 g/kg BW of the animal.

In some embodiments, the components can be administered daily to a canine in the ranges listed in Table 1 on an "as-fed" basis where the components are milligrams per kilogram of the body weight of the canine (mg/kg BW).

**Table 1**

| Component | Range (mg/kg BW) | Range (mg/kg BW) |
|---|---|---|
| Alpha Tocopherol Acetate | 0.1 - 100 | 1 - 50 |
| Vitamin C | 0.1 - 100 | 0.1 - 50 |
| Selenium | 0.001 - 1 | 0.001-0.1 |
| Thiamine | 0.01 - 10 | 0.1 - 1 |
| Riboflavin | 0.01 - 10 | 0.1 - 1 |
| Pantothenic Acid | 0.01 - 10 | 0.1 - 1 |
| Niacin | 0.1 - 100 | 0.1 - 50 |
| Pyridoxine | 0.01 - 10 | 0.1 - 1 |
| Folic Acid | 0.001 - 1 | 0.001 - 0.1 |
| Cyanocobalamin | 0.0001 - 1 | 0.001 - 0.1 |

In some embodiments, the components can be administered daily to a feline in the ranges listed in Table 2 on an "as-fed" basis where the components are milligrams per kilogram of the body weight of the feline (mg/kg BW).

**Table 2**

| Component | Range (mg/kg BW) | Range (mg/kg BW) |
|---|---|---|
| Alpha Tocopherol Acetate | 0.1 - 100 | 1 - 50 |
| Vitamin C | 0.1 - 100 | 0.1 - 50 |
| Selenium | 0.001 - 1 | 0.001 - 0.1 |
| Thiamine | 0.01 - 10 | 0.1 - 1 |
| Riboflavin | 0.01 - 10 | 0.1 - 1 |
| Pantothenic Acid | 0.01 - 10 | 0.1 - 1 |
| Pyridoxine | 0.01 - 10 | 0.1 - 10 |
| Cyanocobalamin | 0.0001 - 1 | 0.001 - 0.1 |
| Folic Acid | 0.001 - 1 | 0.01 - 0.1 |

Only a portion of the arginine consumed by an individual remains available for metabolization to NO. Arginine is metabolized into citrulline and NO via the enzyme nitric oxide synthase (NOS). As much as 60% of ingested arginine is metabolized in the liver by arginase before entering the circulation, where any remaining arginine may be metabolized to citrulline and NO. An alternative source for arginine is the endogenous production of arginine from the amino acid citrulline. This route contributes about 20% to whole body arginine production. Citrulline is produced in the intestine and enters the circulation without being metabolized by the liver, with almost complete conversion to arginine in the kidneys. Citrulline passes through the liver with little or no metabolization and is converted to arginine in the mitochondria. The majority of circulating citrulline is converted in the kidneys, which are comprised of highly metabolically active tissue. Citrulline circulating in the bloodstream is first converted to arginine and then in cells to citrulline and NO. Citrulline can be administered via the gastrointestinal (GI) tract in oral and enteral products. Citrulline, endogenously, is a product of the metabolism of glutamine in the gut, generated from ornithine as part of the urea cycle, and formed by nitric oxide synthases distributed in the body. Arginine is generated from citrulline, mainly in the kidney, through its metabolism by argininosuccinate synthase (EC 6.3.4.5) and argininosuccinate lyase (EC 4.3.2.1). Significantly, the conversion of citrulline to arginine occurs continuously, as long as citrulline is circulating in the bloodstream. As a result, circulating citrulline makes it possible to maintain elevated concentrations of arginine over time, which in turn makes it possible to maintain a steady release of NO in cells. Since the amino acid citrulline is a precursor to L-arginine, citrulline can substitute for L-arginine in nutritional compositions. Unlike arginine, citrulline is not metabolized by the liver following entry into the bloodstream through absorption from the diet or *de novo* intestinal production. Citrulline is enzymatically converted to arginine by mitochondria via a part of the urea cycle.

Similarly, ornithine is a precursor to L-arginine. As a result, ornithine can substitute for L-arginine in nutritional compositions. Unlike arginine, ornithine is not metabolized by the liver following entry into the bloodstream through absorption from the diet or *de novo* intestinal production. Ornithine is enzymatically converted to arginine by mitochondria via a part of the urea cycle.

Administration of the composition for use according to the present invention or in accordance with the methods can be on an as-needed or as-desired basis of varying or regular frequency. A goal of regular ingestion is to provide the animal with a regular and consistent dose of the composition or the direct or indirect metabolites that result from such ingestion. Such regular and consistent dosing will tend to create constant blood levels of the components of the compositions or their direct or indirect metabolites. Thus, regular administration can be once monthly, once weekly, once daily, or more than once daily. Similarly, administration can be every other day, week, or month, every third day, week, or month, every fourth day, week, or month, and the like. Administration can be multiple times per day. When utilized as a supplement to ordinary dietetic requirements, the composition may be administered directly to the animal, e.g., orally, or otherwise. The compositions can alternatively be contacted with, or admixed with, daily feed or food, including a fluid, such as drinking water, or an intravenous connection for an animal that is receiving such treatment. When utilized as a daily feed or food, administration will be well known to those of ordinary skill.

Administration can also be carried out as part of a dietary regimen for the animal. For example, a dietary regimen may comprise causing the regular ingestion by the animal of a composition described herein in an amount effective to enhance neurogenesis in the animal.

According to the compositions for use according to the present invention or the methods of the invention, administration of the compositions, including administration as part of a dietary regimen, can span a period of time ranging from parturition through the adult life of the animal. In various embodiments, the animal can be a human or companion animal such as a dog or cat. In certain embodiments, the animal can be a young or growing animal. In other embodiments, the animal can be an aging animal. An animal that has reached about 35% of its projected lifespan can be included. In some embodiments, administration begins, for example, on a regular or extended regular basis, when the animal has reached more than about 30%, 40%, or 50% of its projected or anticipated lifespan. In some embodiments, the animal can have attained 40, 45, or 50% of its anticipated lifespan. In yet other embodiments, the animal can be older having reached 60, 66, 70, 75, or 80% of its likely lifespan. A determination of lifespan may be based on actuarial tables, calculations, estimates, or the like, and may consider past, present, and future influences or factors that are known to positively or negatively affect lifespan. Consideration of species, gender, size, genetic factors, environmental factors and stressors, present and past health status, past and present nutritional status, stressors, and the like may also influence or be taken into consideration when determining lifespan.

In various embodiments of the compositions for use according to the invention or the methods, the composition can be a human food composition, pet food composition, or a dietary supplement. In other embodiments, the composition can be a food composition further comprising about 15% to about 50% protein, about 5% to about 40% fat, about 5% to about 10% ash content, and having a moisture content of about 5% to about 20%.

In certain embodiments, the UFA can be a fish oil and the NORC can be arginine or a nitric oxide-releasing derivative thereof.

In certain embodiments, the composition being administered comprises from about 0.1% to about 50% fish oil and from about 0.1% to about 20% arginine. For embodiments of the methods, as with the compositions above, the UFA comprises one or more of a natural fish oil, ALA, EPA, DPA, DHA, another polyunsaturated fatty acid from any source, or combinations thereof.

The compositions, after being administered to an animal, are believed to increase the circulating concentrations of UFA and NORC in the blood or blood plasma of the animal. In certain embodiments, the blood concentration of a direct or indirect metabolite that results from the consumption of UFA and/or NORC is increased, and is useful as an indicator of dose. A decrease in concentration of one or more compounds in the bloodstream of an animal receiving the compositions can also occur in the normal course. Such decrease can also be a useful tool for monitoring or determining dosages. In one aspect, the change in amount of the bloodstream component to be measured can be dosage dependent. In some embodiments, the method can result in an increase in one or more ketone bodies in the animal's blood. Administration can also result in an increase in circulating concentrations of any B vitamins and/or antioxidants present and can be useful as an indication of dose.

In a further aspect, the invention provides kits suitable for administering a composition comprising one or more UFA and NORC to an animal. The kits comprise in separate containers in a single package or in separate containers in a virtual package, as appropriate for the kit component, (a) a UFAs, (b) an NORC, (c) a B vitamin, (d) an antioxidant, and one or more of (1) one or more other ingredients suitable for consumption by an animal; (2) one or more cognitive drugs; (3) one or more prebiotics; (4) one or more probiotics; (5) one or more diagnostic devices suitable for determining whether an animal could benefit from compositions and methods for enhancing neuorgenesis; (6) instructions for how to combine or prepare the UFA and NORC and any other ingredients provided in the kit for administration to an animal; (7) instructions for how to use the combined kit components, prepared kit components, or other kit components for the benefit of an animal; and (8) a device for administering the combined or prepared kit components to an animal. The components are each provided in separate containers in a single package or in mixtures of various components in different packages. The kits may comprise the ingredients in various combinations. For example, the kit could comprise a mixture of B vitamins and one or more antioxidants in one container and one or more other ingredients in one or more other containers. Similarly, the kit could comprise a mixture of UFA and NORC in one container and one or more other ingredients in one or more other containers. Other such combinations can be produced by the skilled artisan based upon the characteristics of the ingredients and their physical and chemical properties and compatibilities.

In another aspect, the invention provides a means for communicating information about or instructions for one or more of (1) using compositions of the present invention for enhancing neurogenesis; (2) admixing the UFAs, NORC, B vitamins, antioxidants, or other components of the invention to produce a composition suitable for enhancing neurogenesis; (3) using the kits of the present invention for enhancing neurogenesis; and (4) administering the compositions to an animal. The means comprises one or more of a physical or electronic document, digital storage media, optical storage media, audio presentation, audiovisual display, or visual display containing the information or instructions. In one embodiment, the means can be selected from the group consisting of a displayed website, a visual display kiosk, a brochure, a product label, a package insert, an advertisement, a handout, a public announcement, an audiotape, a videotape, a DVD, a CD-ROM, a computer readable chip, a computer readable card, a computer readable disk, a USB device, a FireWire device, a computer memory, and any combination thereof.

In another aspect, the invention provides methods for manufacturing a food composition comprising UFA, NORC, a B vitamin, and an antioxidant, and one or more other ingredients suitable for consumption by an animal, e.g., protein, fat, carbohydrate, fiber, etc. The methods comprise admixing one or more ingredients suitable for consumption by an animal with UFA and NORC, and possibly other ingredients such as B vitamins and/or antioxidants. Alternatively, the methods comprise applying UFA and NORC, and other ingredients if desired, separately or in any combination onto the food composition, e.g., as a coating or topping. The UFA and NORC can be added at any time during the manufacture and/or processing of the food composition. This includes, for example, admixing the UFA and NORC as part of the core formulation of the "body" of the food composition or applying them as a coating, *i.e.,* primarily to the surface of the food composition after its manufacture. The compositions can be made according to any method suitable in the art.

In another aspect, the present invention provides a package comprising a composition of the present invention and a label affixed to the package containing a word or words, picture, design, acronym, slogan, phrase, or other device, or combination thereof, that indicates that the contents of the package contains a composition suitable for enhancing neurogenesis in an animal, in one aspect, an aging animal. Typically, such device comprises the words "improves neurogenesis", or an equivalent expression printed on the package. Any package or packaging material suitable for containing the composition can be useful in the invention, e.g., a bag, box, bottle, can, pouch, and the like manufactured from paper, plastic, foil, metal, and the like. In a one embodiment, the package can contain a food composition adapted for a particular animal such as a human, canine or feline, as appropriate for the label, in one aspect, a companion animal food composition.

In the invention, the animal can be a juvenile, adult, senior, or geriatric animal. Typically, for most embodiments, the animal can be an aging animal. Generally, animals can be senior in the last half of their expected lifespan and geriatric in the last fourth of their expected lifespan. Lifespan definitions vary for various animals and are known to skilled artisans. For example, a human can be considered to be juvenile until about 16 years of age. For a dog or cat, the animal can be considered to be juvenile until 1 year of age.

The compositions of the invention, including the pharmaceutical compositions and medicaments, can be administered to the animal using a variety of administration routes. Such routes include oral, intranasal, intravenous, intramuscular, intragastric, transpyloric, subcutaneous, rectal, and the like. In one aspect, the compositions can be administered orally.

The compositions of the invention, including the pharmaceutical compositions and medicaments, can be administered to the animal for a time required to accomplish one or more objectives of the invention, *e.g.,* enhancing neurogenesis in an animal. The compositions can be suitable for long-term administration or administration on any schedule compatible with the composition and objective.

In various embodiments of the methods and compositions of the invention, the antioxidants comprise astaxanthin alone or in combination with other antioxidants.

### EXAMPLES

The invention can be further illustrated by the following example, although it will be understood that this example is included merely for purposes of illustration and is not intended to limit the scope of the invention unless otherwise specifically indicated.

### Example 1 - Alzheimer's disease mouse model (AD mice)

This transgenic model over-expresses 5 FAD mutations: both mutant human APP (695) with the Swedish (K670N, M671L), Florida (I716V), and London (V717I) Familial Alzheimer's Disease (FAD) mutations and human PS1 harboring two FAD mutations, M146L and L286V.

All the mice were bred and genotyped and maintained in a temperature-controlled room (22-25°C) with 12-hour dark-light cycles. All mice had free access to diets and tap water during the study period.

### Groups: Intact male mice (4 weeks old)

- Group A: Negative Control: APP PS1 transgenic mice (AD mice), control diet feeding for 11 months.
- Group B: Pre-treatment: APP PS1 transgenic mice (AD mice) + test diet feeding for 11 months (before amyloid plaques).
- Group C: Post-treatment: APP PS1 transgenic mice (AD mice) + control diet feeding 5 months then switch to test diet II for 6 months (after amyloid plaques).
- Group D: Positive control: Non-AD littermate feeding control diet for 11 months.

### Test diets

The Control Diet was the AIN-93M diet containing 140 g/kg casein, 100 g/kg sucrose, 50 g/kg fiber, 155 g/kg dextrin, 466 g/kg corn starch, 35 g/kg standard salt mix, 40 g/kg soybean oil, 10 g/kg standard vitamin mix, 1.8 g/kg L-cystine and 2.5 g/kg choline chloride.

The test diet was the AIN-93M diet supplemented with 2% Arginine, 2% Menhaden fish oil, 4X B vitamins, and antioxidants (Vitamin E: 500 mg/kg diet, Vitamin C: 150 mg/kg diet, Astaxanthin:100 mg/kg, selenium: 0.40 mg/kg). B vitamins included vitamin B1 (thiamine), vitamin B2 (riboflavin), vitamin B3 (niacin or nicotinic acid), vitamin B5 (Pantothenic acid), vitamin B6 (pyridoxine, pyridoxal, pyridoxamine), vitamin B7 (biotin), vitamin B9 (folic acid), and vitamin B12 (cobalamins).

### Neuron density measurement

At the end of experiments, all the mice were allowed to recover for two weeks after they finish all the behavior tests. Then, all animals were sacrificed by necropsy. Hemi-brains were harvested and post fixed in 4% formalin, then processed for paraffin embedding. Using a microtome serial 6 micron thick sagittal sections were cut. Starting from the sagittal surface approximately 180 microns were cut and discarded till hippocampus was visible. Once hippocampus was visible sections were collected on a glass slide. Slides were dried overnight at 37 °C and were process for H&E staining. Then, images were taken under 20X objective using Carl Ziess microscope.

Cells were counted in similar hippocampal sections from each mouse using Image J software. Each section was from a matched area of the brain and the sections scored were from similar areas in the CA1 region. A cell of representative size was chosen and "circled" using the Image J program. The cell was then selected and numbered and its area recorded. The "circle" was then moved to another cell and that cell was labeled and its area recorded. This continued until all the cells in the hippocampus were recorded. Only cells with a nucleus were recorded and cells of a noticeably smaller size were also not counted. The personnel conducting cell count was blind to phenotype and treatment condition.

**Table 3. Neuron density (# of neuron/area)**

| | AD mice + control diet | AD mice + test diet for 11M | AD mice + test diet for 6M | Non-AD mice + control diet |
|---|---|---|---|---|
| Mean | 115.0 | 162.1 | 135.5 | 121.9 |

The test diets increase neuron numbers at the hippocampal CA1 region in AD mice, indicating that the blend enhances neurogenesis in adult animals. With the non-AD mice fed the control diet as the positive control for the normal level of neuron density of mice at the age of 12 months old, the results show an unexpected discovery that after a chronic feeding of a nutrient blend including fish oil, arginine, B vitamins and antioxidants, neuron density was increased in CA1 region of the hippocampus in a mouse model of Alzheimer's disease (AD mice), indicating that the blend is able to stimulate adult neurogenesis at CA1 region of the hippocampus. Further, when the test diet was administered to young, growing mice, the neurogenesis effect was significantly higher than when fed after reaching adulthood (compare neuron density of 162.1 to 135.5 in Table 3). Perhaps more importantly, the control diet containing adequate levels of arginine, B vitamins, and antioxidants failed to enhance neuron density at CA1 in the AD mice. In fact, AD mice fed the control diet tended to have lower neuron density compared with the non-AD mice fed the control diet. These data indicate that a nutrient blend containing fish oil, arginine, B vitamins and antioxidants enhance adult neurogenesis at CA1 of hippocampus, while adequate amounts of arginine, B vitamins, and antioxidants in the control diet fails to enhance neurogenesis at CA1.

## Claims

1. A composition for use in enhancing neurogenesis in an animal that is suffering from cognitive decline, that is at risk of becoming cognitively impaired, that is diagnosed with a disease, disorder, or other condition that affects its cognitive abilities, or that is at risk of contracting a disease, disorder, or other condition that affects its cognitive abilities, the composition comprising an unsaturated fatty acid (UFA), a nitric oxide releasing compound (NORC), a B vitamin, and an antioxidant, in an amount effective for enhancing neurogenesis in the animal.

2. The composition for the use of claim 1, wherein the neurogenesis occurs in the hippocampus in the animal and the neuron density is increased by at least 10%.

3. The composition for the use of claim 1, wherein the composition comprises from about 0.1% to about 50% UFA and wherein the UFA comprises one or more of a natural fish oil, ALA, EPA, DPA, DHA, or another n-3 fatty acid from any source and/or
wherein the composition comprises from about 0.1% to about 20% NORC and wherein the NORC is arginine, citrulline, ornithine, or a nitric oxide-releasing derivative thereof and/or wherein the composition comprises from about 1,2 to 40 times the recommended daily requirement of the B vitamin and wherein the B vitamin is selected from the group consisting of vitamin B1, vitamin B2, vitamin B3, vitamin B5, vitamin B6, vitamin B7, vitamin B8, vitamin B9, vitamin B12, and mixtures thereof and/or
wherein the composition comprises from about 0.0001% to about 25% of the antioxidant and wherein the antioxidant is selected from any of the group consisting of carotenoids, flavonoids, phenolic acids and their esters, nonflavonoid phenolics, curcumin, flavonolignans, xanthones, eugenol, vitamin C, vitamin E, selenium, and mixtures thereof.

4. The composition for the use of claim 1, wherein the composition is formulated as a human food composition, pet food composition, or a dietary supplement.

5. The composition for the use of claim 1, wherein the animal has a phenotype associated with age-related cognitive impairment, wherein the phenotype includes one or more of decreased ability to recall, short-term memory loss, decreased learning rate, decreased capacity for learning, decreased problem solving skills, decreased attention span, decreased motor performance, increased confusion, or dementia, as compared to a control animal not having the phenotype.

6. The composition for the use of claim 1, wherein the animal is a canine or a feline.

7. A non-therapeutic method for enhancing neurogenesis in a growing animal comprising: identifying the growing animal, and administering a composition comprising an unsaturated fatty acid (UFA), a nitric oxide releasing compound (NORC), a B vitamin, and an antioxidant, to the growing animal in an amount effective for enhancing neurogenesis in the growing animal.

8. The non-therapeutic method of claim 7, wherein the neurogenesis occurs in the hippocampus in the growing animal and the neuron density is increased by at least 10%.

9. The non-therapeutic method of claim 7, wherein the composition comprises from about 0.1% to about 50% UFA and wherein the UFA comprises one or more of a natural fish oil, ALA, EPA, DPA, DHA, or another n-3 fatty acid from any source and/or
wherein the composition comprises from about 0.1% to about 20% NORC and wherein the NORC is arginine, citrulline, ornithine, or a nitric oxide-releasing derivative thereof and/or
wherein the composition comprises from about 1,2 to 40 times the recommended daily requirement of the B vitamin and wherein the B vitamin is selected from the group consisting of vitamin B1, vitamin B2, vitamin B3, vitamin B5, vitamin B6, vitamin B7, vitamin B8, vitamin B9, vitamin B12, and mixtures thereof and/or
wherein the composition comprises from about 0.0001%to about 25% of the antioxidant and wherein the antioxidant is selected from any of the group consisting of carotenoids, flavonoids , phenolic acids and their esters, nonflavonoid phenolics, curcumin, flavonolignans, xanthones, eugenol, vitamin C, vitamin E, selenium, and mixtures thereof.

10. The non-therapeutic method of claim 7, wherein the composition is formulated as a human food composition, pet food composition, or a dietary supplement.

11. The non-therapeutic method of claim 7, wherein the growing animal has reached a life expectancy between 0 and 30%.

12. The non-therapeutic method of claim 7, wherein the growing animal is an infant, a kitten, or a puppy.

13. The non-therapeutic method of claim 7, wherein the composition includes arginine, fish oil, B vitamins, vitamin E, vitamin C, astaxanthin, and selenium.

## Patentansprüche

1. Zusammensetzung zur Verwendung beim Verstärken der Neurogenese bei einem Lebewesen, das an geistigem Abbau leidet, bei dem das Risiko besteht, geistig eingeschränkt zu werden, bei dem eine Krankheit, Störung oder ein anderer Zustand diagnostiziert wurde, die/der seine geistigen Fähigkeiten beeinträchtigt, oder bei dem das Risiko besteht, das es an einer Krankheit, Störung oder einem anderen Zustand erkrankt, die/der seine geistigen Fähigkeiten beeinträchtigt, wobei die Zusammensetzung eine ungesättigte Fettsäure (UFA), eine stickstoffmonoxidfreisetzende Verbindung (NORC), ein B-Vitamin und ein Antioxidans in einer Menge umfasst, die zum Verstärken der Neurogenese bei dem Lebewesen wirksam ist.

2. Zusammensetzung zu der Verwendung nach Anspruch 1, wobei die Neurogenese bei dem Lebewesen in dem Hippocampus auftritt und die Neuronendichte um mindestens 10 % erhöht wird.

3. Zusammensetzung zu der Verwendung nach Anspruch 1, wobei die Zusammensetzung etwa 0,1 % bis etwa 50 % UFA umfasst und wobei die UFA eines oder mehrere von einem natürlichen Fischöl, ALA, EPA, DPA, DHA oder einer anderen n-3-Fettsäure aus einer beliebigen Quelle umfasst und/oder wobei die Zusammensetzung etwa 0,1 % bis etwa 20 % NORC umfasst und wobei die NORC Arginin, Citrullin, Ornithin oder ein stickstoffmonoxidfreisetzendes Derivat davon umfasst und/oder wobei die Zusammensetzung das etwa 1,2- bis 40-fache des empfohlenen Tagesbedarfs des B-Vitamins umfasst und wobei das B-Vitamin aus der Gruppe ausgewählt ist, die aus Vitamin B1, Vitamin B2, Vitamin B3, Vitamin B5, Vitamin B6, Vitamin B7, Vitamin B8, Vitamin B9, Vitamin B12 und Mischungen davon besteht, und/oder
wobei die Zusammensetzung etwa 0,0001 % bis etwa 25 % des Antioxidans umfasst und wobei das Antioxidans aus einem beliebigen der Gruppe ausgewählt ist, die aus Carotinoiden, Flavonoiden, Phenolsäuren und deren Estern, Nonflavonoidphenolen, Curcumin, Flavonolignanen, Xanthonen, Eugenol, Vitamin C, Vitamin E, Selen und Mischungen davon besteht.

4. Zusammensetzung zu der Verwendung nach Anspruch 1, wobei die Zusammensetzung als eine Lebensmittelzusammensetzung für Menschen, Lebensmittelzusammensetzung für Haustiere oder ein Nahrungsergänzungsmittel formuliert ist.

5. Zusammensetzung zu der Verwendung nach Anspruch 1, wobei das Lebewesen einen Phänotypus aufweist, der mit einer altersbedingten geistigen Einschränkung in Zusammenhang steht, wobei der Phänotypus eines oder mehrere von verminderter Erinnerungsfähigkeit, Kurzzeitgedächtnisverlust, verminderter Lerngeschwindigkeit, verminderter Lernkapazität, verminderten Problemlösefähigkeiten, verminderter Aufmerksamkeitsspanne, verminderter motorischer Leistung, vermehrter Verwirrung oder Demenz, verglichen mit einem Kontrolllebewesen, das den Phänotypus nicht aufweist, einschließt.

6. Zusammensetzung zu der Verwendung nach Anspruch 1, wobei das Lebewesen ein Hund oder eine Katze ist.

7. Nichttherapeutisches Verfahren zum Verstärken der Neurogenese bei einem wachsenden Lebewesen, umfassend: Identifizieren des wachsenden Lebewesens und Verabreichen einer Zusammensetzung, umfassend eine ungesättigte Fettsäure (UFA), eine stickstoffmonoxidfreisetzende Verbindung (NORC), ein B-Vitamin und ein Antioxidans, an das wachsende Lebewesen in einer Menge, die zum Verstärken der Neurogenese bei dem wachsenden Lebewesen wirksam ist.

8. Nichttherapeutisches Verfahren nach Anspruch 7, wobei die Neurogenese in dem Hippocampus bei dem wachsenden Lebewesen auftritt und die Neuronendichte um mindestens 10 % erhöht wird.

9. Nichttherapeutisches Verfahren nach Anspruch 7, wobei die Zusammensetzung etwa 0,1 % bis etwa 50 % UFA umfasst und wobei die UFA eines oder mehrere von natürlichem Fischöl, ALA, EPA, DPA, DHA oder einer anderen n-3-Fettsäure aus einer beliebigen Quelle umfasst und/oder
wobei die Zusammensetzung etwa 0,1 % bis etwa 20 % NORC umfasst und wobei die NORC Arginin, Citrullin, Ornithin oder ein stickstoffmonoxidfreisetzendes Derivat davon ist und/oder
wobei die Zusammensetzung das etwa 1,2- bis 40-fache des empfohlenen Tagesbedarfs des B-Vitamin umfasst und wobei das B-Vitamin aus der Gruppe ausgewählt ist, die aus Vitamin B1, Vitamin B2, Vitamin B3, Vitamin B5, Vitamin B6, Vitamin B7, Vitamin B8, Vitamin B9, Vitamin B12 und Mischungen davon besteht, und/oder
wobei die Zusammensetzung etwa 0,0001 % bis etwa 25 % des Antioxidans umfasst und wobei das Antioxidans aus einem beliebigen der Gruppe ausgewählt ist, die aus Carotinoiden, Flavonoiden, Phenolsäuren und deren Estern, Nonflavonoidphenolen, Curcumin, Flavonolignanen, Xanthonen, Eugenol, Vitamin C, Vitamin E, Selen und Mischungen davon besteht.

10. Nichttherapeutisches Verfahren nach Anspruch 7, wobei die Zusammensetzung als eine Lebensmittelzusammensetzung für Menschen, Lebensmittelzusammensetzung für Haustiere oder ein Nahrungsergänzungsmittel formuliert ist.

11. Nichttherapeutisches Verfahren nach Anspruch 7, wobei das wachsende Lebewesen eine Lebenserwartung von zwischen 0 und 30 % erreicht hat.

12. Nichttherapeutisches Verfahren nach Anspruch 7, wobei das wachsende Lebewesen ein Säugling, ein Kätzchen oder ein Welpe ist.

13. Nichttherapeutisches Verfahren nach Anspruch 7, wobei die Zusammensetzung Arginin, Fischöl, B-Vitamine, Vitamin E, Vitamin C, Astaxanthin und Selen umfasst.

## Revendications

1. Composition pour une utilisation pour améliorer la neurogenèse chez un animal qui souffre de déclin cognitif, qui risque de devenir cognitivement déficient, qui est diagnostiqué avec une maladie, un trouble ou une autre affection qui affecte ses aptitudes cognitives, ou qui risque de contracter une maladie, un trouble ou une autre affection qui affecte ses capacités cognitives, la composition comprenant un acide gras insaturé (AGI), un composé libérant de l'oxyde nitrique (CLON), une vitamine B et un antioxydant, en une quantité efficace pour améliorer la neurogenèse chez l'animal.

2. Composition pour l'utilisation selon la revendication 1, dans laquelle la neurogenèse se produit dans l'hippocampe chez l'animal et la densité de neurones est augmentée d'au moins 10 %.

3. Composition pour l'utilisation selon la revendication 1, dans laquelle la composition comprend d'environ 0,1 % à environ 50 % d'AGI et dans laquelle l'AGI comprend un ou plusieurs parmi une huile naturelle de poisson, ALA, EPA, DPA, DHA, ou un autre acide gras n-3 provenant de n'importe quelle source et/ou dans laquelle la composition comprend d'environ 0,1 % à environ 20 % de CLON et dans laquelle le CLON est arginine, citrulline, ornithine, ou un dérivé libérant de l'oxyde nitrique de celles-ci et/ou dans laquelle la composition comprend d'environ 1,2 à 40 fois l'apport quotidien recommandé de la vitamine B et dans laquelle la vitamine B est choisie dans le groupe constitué de vitamine B1, vitamine B2, vitamine B3, vitamine B5, vitamine B6, vitamine B7, vitamine B8, vitamine B9, vitamine B12, et des mélanges de celles-ci et/ou
dans laquelle la composition comprend d'environ 0,0001 % à environ 25 % de l'antioxydant et dans laquelle l'antioxydant est choisi parmi n'importe lequel du groupe constitué de caroténoïdes, flavonoïdes, acides phénoliques et leurs esters, composés phénoliques non-flavonoïdes, curcumine, flavonolignanes, xanthones, eugénol, vitamine C, vitamine E, sélénium, et des mélanges de ceux-ci.

4. Composition pour l'utilisation selon la revendication 1 dans laquelle la composition est formulée en tant que composition alimentaire humaine, composition alimentaire pour animaux domestiques, ou complément alimentaire.

5. Composition pour l'utilisation selon la revendication 1, dans laquelle l'animal a un phénotype associé à un déclin cognitif lié à l'âge, dans laquelle le phénotype inclut une ou plusieurs parmi une aptitude diminuée à se souvenir, une perte de mémoire à court terme, une vitesse d'apprentissage réduite, une aptitude d'apprentissage affaiblie, une diminution des aptitudes à résoudre les problèmes, une aptitude de concentration diminuée, une diminution de la performance motrice, une confusion accrue ou la démence, par rapport à un animal témoin n'ayant pas le phénotype.

6. Composition pour l'utilisation selon la revendication 1, dans laquelle l'animal est un canidé ou un félidé.

7. Procédé non thérapeutique pour améliorer la neurogenèse chez un animal en croissance comprenant : l'identification de l'animal en croissance, et l'administration d'une composition comprenant un acide gras insaturé (AGI), un composé libérant de l'oxyde nitrique (CLON), une vitamine B, et un antioxydant, à l'animal en croissance en une quantité efficace pour améliorer la neurogenèse chez l'animal en croissance.

8. Procédé non thérapeutique selon la revendication 7, dans lequel la neurogenèse se produit dans l'hippocampe chez l'animal en croissance et la densité de neurones est augmentée d'au moins 10 %.

9. Procédé non thérapeutique selon la revendication 7, dans lequel la composition comprend d'environ 0,1 % à environ 50 % d'AGI et dans lequel l'AGI comprend un ou plusieurs parmi une huile naturelle de poisson, ALA, EPA, DPA, DHA, ou un autre acide gras n-3 provenant de n'importe quelle source et/ou
dans lequel la composition comprend d'environ 0,1 % à environ 20 % de CLON et dans lequel le CLON est arginine, citrulline, ornithine, ou un dérivé libérant de l'oxyde nitrique de celles-ci et/ou
dans lequel la composition comprend d'environ 1,2 à 40 fois l'apport quotidien recommandé de la vitamine B et dans lequel la vitamine B est choisie dans le groupe constitué de vitamine B1, vitamine B2, vitamine B3, vitamine B5, vitamine B6, vitamine B7, vitamine B8, vitamine B9, vitamine B12, et des mélanges de celles-ci et/ou
dans lequel la composition comprend d'environ 0,0001 % à environ 25 % de l'antioxydant et dans lequel l'antioxydant est choisi parmi n'importe lequel du groupe constitué de caroténoïdes, flavonoïdes, acides phénoliques et leurs esters, composés phénoliques non-flavonoïdes, curcumine, flavonolignanes, xanthones, eugénol, vitamine C, vitamine E, sélénium, et des mélanges de ceux-ci.

10. Procédé non thérapeutique selon la revendication 7, dans lequel la composition est formulée en tant que composition alimentaire humaine, composition alimentaire pour animaux domestiques, ou complément alimentaire.

11. Procédé non thérapeutique selon la revendication 7, dans lequel l'animal en croissance a atteint une espérance de vie entre 0 et 30 %.

12. Procédé non thérapeutique selon la revendication 7, dans lequel l'animal en croissance est un nourrisson, un chaton, ou un chiot.

13. Procédé non thérapeutique selon la revendication 7, dans lequel la composition inclut de l'arginine, de l'huile de poisson, des vitamines B, de la vitamine E, de la vitamine C, de l'astaxanthine, et du sélénium.
